# NEUE EUROPÄISCHE PATENTSCHRIFT

(11) **EP 0 730 853 B2**
(45) Veröffentlichungstag und Bekanntmachung des Hinweises auf die Entscheidung über den Einspruch: **22.05.2002**
(45) Hinweis auf die Patenterteilung: 28.04.1999
(21) Anmeldenummer: 96102151.6
(22) Anmeldetag: 14.02.1996
(51) Int. Cl.: A61F 17/00

(54) **Nothilfepackung**
First-aid kit
Trousse de premier secours

(30) Priorität: 25.02.1995 DE 29503188 U
(43) Veröffentlichungstag der Anmeldung: 11.09.1996
(73) Patentinhaber: FRANZ KALFF GmbH, D-53881 Euskirchen (DE)
(72) Erfinder: Eichhorn Wilfried, D-61273 Wehrheim (DE)
(74) Vertreter: Selting, Günther, Dipl.-Ing.

(56) Entgegenhaltungen:
- DE-C- 880 192
- DE-U- 9 400 932
- DE-U- 29 515 306
- FR-A- 1 095 236
- FR-A- 1 196 257
- FR-A- 2 545 366
- GB-A- 2 202 428
- JP-A- 568 696
- JP-A- 5 068 696
- US-A- 4 241 833

## Beschreibung

Die Erfindung bezieht sich auf eine Nothilfepackung, bestehend aus einer flexiblen, länglichen Materialbahn, welche in Querabschnitte unterteilt ist, die Taschen zur Aufnahme von Erste-Hilfe-Produkten aufweisen und alle zickzackförmig aufeinandergestapelt sind.

Eine Nothilfepackung ist bekannt aus FR-A-1 095 236. Diese Nothilfepackung besteht aus einem Behältnis, das nach Art eines Buchdeckels aufgeklappt werden kann und in der sich eine zickzackförmig gefaltete doppellagige Einlage befindet. Die Einlage bildet Taschen, die hermetisch abgeschlossen und umfangsmäßig verschweißt sind und die aufzubewahrenden Gegenstände enthalten. Bei einer derartigen Nothilfepackung müssen die Taschen aufgerissen werden, um den Inhalt entnehmen zu können. Hierzu sind entsprechende Aufreißkerben vorgesehen.

Aus DE-U-80 02 838 ist eine Nothilfepackung bekannt, bei der die Querabschnitte einer flexiblen, länglichen Materialbahn ausgehend von einem Ende durch rollende Zusammenlegung zu einem Wickel gebündelt werden, der durch Verschlußmittel zusammengehalten ist. Zugang zum Inhalt der Taschen erhält man durch Auseinanderrollen des Materialbahnwickels, wozu beide Hände benötigt werden. Dieser Vorgang wird insbesondere dann erschwert, wenn keine saubere Unterlage ausreichender Größe für die flachgelegte Materialbahn vorhanden ist, was im Notfalle, zum Beispiel bei einem Verkehrsunfall, durchaus die Regel ist.

Eine andere Nothilfepackung, von der der Oberbegriff des Anspruchs 1 ausgeht, ist aus DE-U-94 00 932 bekannt. Auch hierbei wird eine flexible, längliche Materialbahn mit Taschen an Querabschnitten durch Zusammenwickeln zu einem Wickelbündel umgestaltet. Das Wickelbündel ist lose in einen steifen Kasten einlegbar. Zur Entnahme von Gegenständen aus einer Tasche muß das Wikkelbündel aus dem Kasten herausgenommen und außerhalb desselben mit beiden Händen so weit wie nötig auseinandergerollt werden. Die Notwendigkeit der Auflage der flachen Materialbahn auf eine geeignete Unterlage besteht auch bei dieser bekannten Nothilfepackung und auch hierbei kann durch Umständlichkeit der Handhabung der Nothilfepackung wertvolle Zeit bis zur Hilfeleistung verstreichen.

Der Erfindung liegt die Aufgabe zugrunde, eine Nothilfepackung mit flexibler, länglicher Materialbahn so zu verbessern, daß sie mit einer Hand aus der Bündelformation in flachgestreckten Zustand überführbar ist.

Diese Aufgabe wird erfindungsgemäß dadurch gelöst, daß die Querabschnitte zickzackförmig gestapelt sind. Mit Zickzackförmig ist gemeint, daß alle Querabschnitte Zickzackförmig gestapelt sind.

Eine derartige Nothilfepackung bildet ein auf dem untersten Querabschnitt liegendes Faltbündel aus Querabschnitten, das durch Erfassung der obersten Lage des Stapels einhändig auseinandergezogen werden kann. Dabei hängt die gestreckte längliche Materialbahn an der sie haltenden Hand und mit der anderen Hand kann der gesuchte Gegenstand aus einer Tasche entnommen werden. Nach der Entnahme wird die längliche Materialbahn in

Zickzackverlegung in ein Faltbündel zurückverwandelt und nimmt Blockform an. Die Blockform des Faltbündels läßt sich durch beliebige Befestigungsmittel fixieren. Ein solches Faltbündel kann in eine passende Fahrzeugmulde eingelegt und durch einen fahrzeugseitigen Deckel geschützt werden.

Zweckmäßigerweise ist der Querabschnitt der untersten Lage des Stapels an einer Basis lösbar befestigt. Die Basis hält das untere Ende des Stapels beim Auseinanderziehen der Materialbahn fest, wodurch die Handhabung vereinfacht wird. Sie kann in eine Fahrzeugmulde eingelassen sein und verankert die unterste Lage des Stapels in der Fahrzeugmulde, so daß die auseinandergezogene Materialbahn zwischen Hand und Fixpunkt waagerecht gehalten und die Entnahme von Erste-Hilfe-Produkten aus den Taschen erleichtert wird. Die Lösbarkeit des Querabschnittes der untersten Lage des Stapels von der Basis ist günstig, weil die Materialbahn mit Inhalt insgesamt ausgetauscht werden kann, wenn Verfallsdaten der Erste-Hilfe-Produkte überschritten wurden oder die Materialbahn selbst Schaden genommen hat. Die Neuanschaffung bezieht sich dann nur auf die Materialbahn mit Inhalt und die in die Fahrzeugmulde passende Basis braucht nicht ersetzt zu werden.

Die Basis kann der Boden eines viereckigen Kastens mit Seitenwandung sein, wobei die Seitenwandung einen Ausschnitt zum Durchlaß der Materialbahn aufweist. Der Kasten erlaubt eine Einhandbedienung der Nothilfepakkung, weil sich die am Handgriff des obersten Querabschnittes des Stapels erfaßte Materialbahn aus dem Kasten sukzessive entfalten läßt, ohne insgesamt aus diesem herausgenommen werden zu müssen. Der Kasten bleibt beispielsweise auf einem Fahrzeugsitz stehen und durch den Ausschnitt in der Seitenwandung wird die Materialbahn waagerecht vorgezogen. Wenn die Materialbahn voll entfaltet ist, kann sie hingelegt werden, ohne daß der Kasten umkippt, denn die beschwerte Materialbahn, deren letzter (unterster) Querabschnitt des Stapels mit dem Boden des Kastens verbunden bleibt, übt über den niedrigen Seitenwandteil unterhalb des Ausschnittes kein Kippmoment auf den Kasten aus. Die durch den Ausschnitt erleichterte Herausnehmbarkeit der Nothilfepackung aus dem Kasten beschleunigt nicht nur die Hilfeleistung im Notfalle, sondern begünstigt auch eine periodische Über-prüfung des Inhaltes und seiner Verfallsdaten, wodurch gewährleistet ist, daß die Erste-Hilfe-Produkte immer zuverlässig einsatzbereit sind.

Der Kasten weist vorzugsweise einen Klappdeckel auf, dessen Klappachse dem Ausschnitt der Seitenwandung gegenüberliegt, und es ist vorgesehen, daß der Klappdekkel mit einem Leistenteil versehen ist, der den Ausschnitt verschließt. Auf diese Weise ist die Nothilfepackung in dem Kasten geschützt untergebracht. Vorzugsweise ist der Kasten mit einem Einhand-Bedienungsverschluß versehen.

In weiterer vorteilhafter Ausgestaltung der Erfindung kann die Basis Teil eines Verpackungsbeutels sein. Infolge der Integration der Basis in einen Kasten oder einen Verpackungsbeutel bilden diese mit der lösbar an der Basis befestigbaren Materialbahn eine Einheit, die im Normalfall zusammengehalten ist. Kasten und Verpackungsbeutel können für jede Art von Fahrzeug einschließlich Fahrrad konzipiert und stehend/liegend oder hängend montierbar bzw. tragbar sein.

Zur Verhinderung des Herausfallens der Erste-Hilfe-Produkte aus den Taschen während des Zusammen- oder Auseinanderfaltens der Materialbahn ist es günstig, die Taschen auf den beiden Materialbahn-Längshälften anzuordnen und die Öffnungen der Taschen etwa parallel zur Längsmittellinie der Materialbahn verlaufen zu lassen. Die Öffnungen sind einander zugewandt, d. h. die Zugänge zu den Taschen befinden sich beiderseits der Längsmittellinie der Materialbahn.

Der Handgriff ist vorteilhafterweise als Griffloch in einer Materialbahnlasche des Querabschnittes der obersten Lage des Stapels ausgebildet. Diese Integration des Handgriffes in die Materialbahn ist herstellungsmäßig einfach und es sind Überstände über die Materialbahn vermieden, so daß das Faltbündel von glatten Flächen umschlossen und passend in den Kasten einfügbar ist.

In der Zeichnung ist ein Ausführungsbeispiel der Erfindung schematisch dargestellt. Es zeigt:

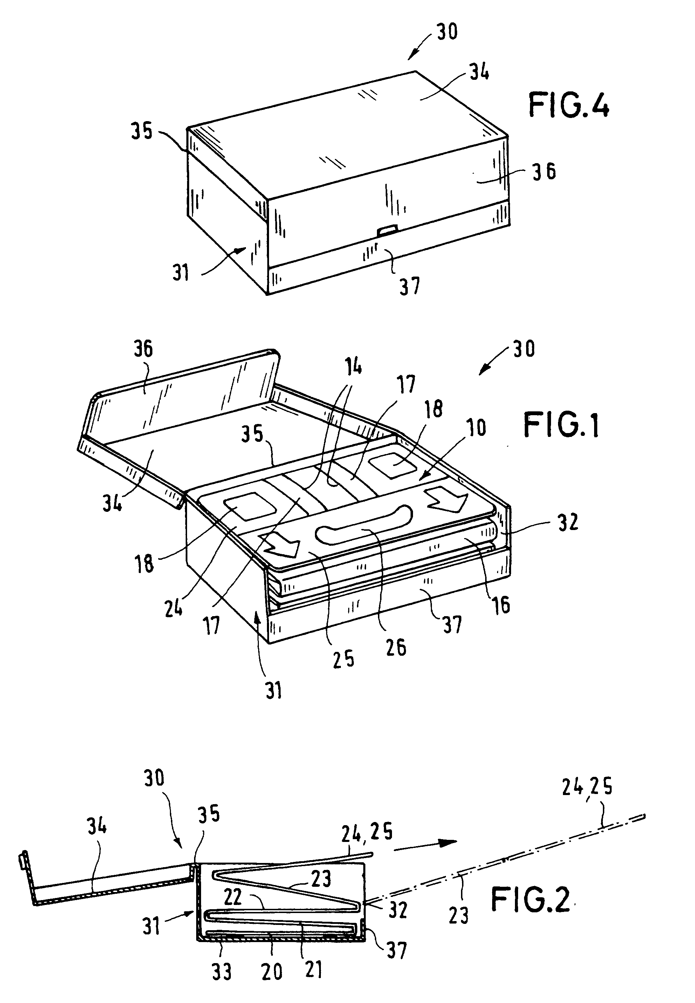

- Fig. 1: einen geöffneten Kasten mit eingelegtem Faltbündel der Nothilfepackung,
- Fig. 2: den geöffneten Kasten in geschnittener Seitenansicht während der Herausziehung der Nothilfepackung,

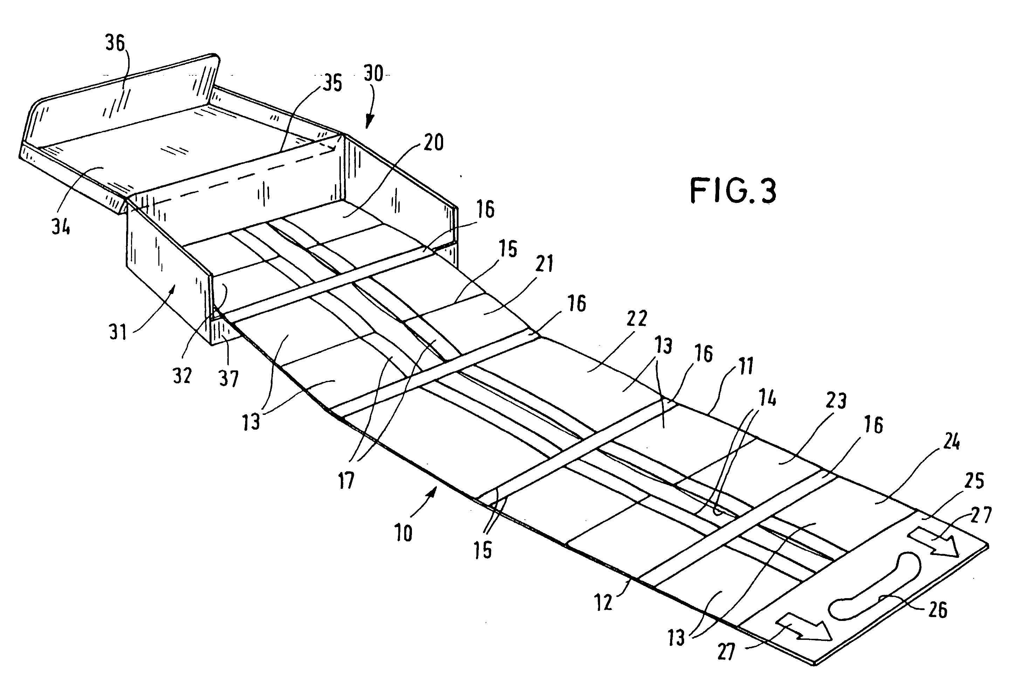

- Fig. 3: eine perspektivische Ansicht der mit dem Querabschnitt der untersten Lage in dem geöffneten Kasten festgelegten und im übrigen vollständig aus diesem herausgezogenen Nothilfepackung, und
- Fig. 4: eine perspektivische Ansicht des geschlossenen Kastens.

Eine Nothilfepackung 10 besteht im wesentlichen aus einer flexiblen, länglichen Materialbahn 11, die aus einer durchgehenden unteren Trägerfolie 12 und mehreren Taschen 13 gebildet ist, die sich auf einer Seite der Trägerfolie 12 befinden. Die Taschen 13 sind auf den beiden Längshälften der Trägerfolie 12 angeordnet und ihre Öffnungen 14 sind der Längsmittellinie der Materialbahn 11 zugewandt und haben zu dieser einen Abstand von vorzugsweise etwa 20 mm. Schweißnähte 15 dienen der Taschenbildung und der Abteilung von schmalen Zwischenzonen 16, die die Materialbahn 11 in Querabschnitte 20, 21,22,23,24 unterteilen, die durch zickzackförmige Faltung zu einem Faltbündel aufeinanderlegbar sind.

Die Zwischenzonen 16 zwischen den Querabschnitten 20 bis 24 erlauben eine einfache Aufeinanderlegung der Querabschnitte 20 bis 24 und haben zudem eine gewisse Buchrückenfunktion, die Quetschungen des Tascheninhaltes bei zusammengelegter Materialbahn 11 verhindern, weil keine scharfen Knicke an den Umlenkstellen entstehen.

Die Querabschnitte 20 bis 24 haben gleiche axiale Länge und sie sind jeweils mit einer beliebigen Anzahl von Taschen 13 ausgerüstet, wobei sich die Beliebigkeit auch auf die Anzahl von Taschen in dem jeweiligen Bereich der Materialbahn-Längshälfte des betreffenden Querabschnittes 20 bis 24 bezieht. Die Taschen 13 sind vorzugsweise so bemessen, daß sie Mullbinden, Kompressen, Verbandtücher, Schnellverbände, Dreiecktücher, Heftpflaster, Sicherheitsnadeln und dergleichen passend aufnehmen, so daß diese in der Tasche 13 festliegen.

Die Trägerfolie 12 ist vorzugsweise undurchsichtig, während die Wände der Taschen 13 zumindest teilweise transparent sind. Die Wände der Taschen 13 sind entlang eines öffnungsseitigen Streifens 17 dunkel gefärbt und mit Angaben zur Zweckbestimmung des Tascheninhaltes versehen. Außerdem weist die Wand jeder Tasche 13 ein undurchsichtiges Feld 18 mit einem Piktogramm auf, das zum Beispiel die Anwendung des Inhaltes der jeweiligen Taschen 13 symbolisiert. Das Feld 18 bedeckt nur einen Teil der Wandfläche, so daß der Inhalt der Tasche 13 durch die transparente Wand hindurch erkennbar ist.

In gestapeltem Zustand der Materialbahn 11 liegt der Querabschnitt 20 unten und der Querabschnitt 24 bildet die oberste Lage des Stapels. Der Querabschnitt 24 ist nur auf etwa der Hälfte seiner axialen Länge mit Taschen 13 besetzt; die andere Hälfte ist als doppellagige Materialbahnlasche 25 gestaltet, die sich über die gesamte Breite der Materialbahn 11 erstreckt. In der Mitte der Materialbahnlasche 25 ist ein Griffloch 26 ausgebildet, das als Handgriff dient und eine Langstreckung des zickzackförmig gelegten Faltbündels durch Ziehen in Richtung der Pfeile 27 auf einfache Weise erlaubt.

Die zickzackförmig zusammengefaltete Materialbahn 11 ist in einem steifen Kasten 30 untergebracht, der aus Kunststoff bestehen kann. Der Hohlraum des quaderförmigen Kastens 30 ist der Abmessung der zusammengelegten Materialbahn 11 im wesentlichen angepaßt, so daß das Faltbündel verschiebungssicher in dem Kasten 30 liegt. Eine Seitenwandung 31 des Kastens 30 umschließt seinen Hohlraum an drei Seiten in gleichmäßiger Höhe; an der vierten Seite ist sie mit einem Ausschnitt 32 versehen, der sich weit bis zum Boden 33 des Kastens 30 erstreckt, so daß nur noch ein niedriger unterer Wandteil 37 stehenbleibt. Der Ausschnitt 32 ist rechteckig und seine Breite entspricht der Breite der Materialbahn 11, d. h. er schließt sich direkt an die beiden Seitenteile der Seitenwandung 31 an. Zum Verschluß des Kastens 30 dient ein Klappdeckel 34, der an dem dem Ausschnitt 32 gegenüberliegenden hinteren Rand der Seitenwandung 31 über eine Klappachse 35 befestigt ist. Am vorderen Rand des Klappdeckels 34 befindet sich ein Leistenteil 36, dessen Höhe und Breite der Höhe und Breite des Ausschnittes 32 in der Seitenwandung 31 im wesentlichen entspricht, so daß bei geschlossenem Klappdeckel 34 auch der Ausschnitt 32 verschlossen ist (Fig. 4).

Der im Stapel unterste Querabschnitt 20 ist auf dem Boden 33 des Kastens 30 lösbar befestigt. Zu diesem Zweck können der Boden 33 und die Unterseite des Querabschnittes 20 Klettbänder oder -punkte aufweisen oder es können klebende Haftmittel verwendet werden, die lösbar sind. Die Befestigung hat den Vorteil, daß beim Herausziehen der zickzackförmig gefalteten Materialbahn 11 aus dem Kasten 30 Materialbahn 11 und Kasten 30 miteinander verbunden bleiben und der Kasten 30 das eine Ende der Materialbahn 11 während des Streckvorganges festhält, so daß die an dem Griffloch 26 gehaltene Materialbahn 11 sich in waagerechte oder schräge Position bringen läßt, in der ein guter Zugriff zu dem Inhalt der Taschen 13 ermöglicht wird.

## Patentansprüche

1. Nothilfepackung, bestehend aus einer flexiblen, länglichen Materialbahn (11), welche in Querabschnitte (20-24) unterteilt ist, die Taschen (13) zur Aufnahme von Erste-Hilfe-Produkten aufweisen,
wobei die Taschen (13) auf den beiden Materialbahn-Längshälften angeordnet sind und wobei die Öffnungen (14) der Taschen (13) etwa parallel zur Längsmittellinie der Materialbahn (11) verlaufen und einander zugewandt sind, **dadurch gekennzeichnet, , dass** alle Querabschnitte (20 - 24) zickzackförmig aufeinandergestapelt sind.

2. Nothilfepackung nach Anspruch 1, **dadurch gekennzeichnet, daß** das freie Ende des Querabschnittes (24) der obersten Lage des Stapels mit einem Handgriff versehen ist.

3. Nothilfepackung nach Anspruch 2, **dadurch gekennzeichnet, daß** der Querabschnitt (24) der obersten Lage des Stapels eine Materialbahnlasche (25) aufweist, in der sich ein Griffloch (26) befindet.

4. Nothilfepackung nach einem der Ansprüche 1 bis 3, **dadurch gekennzeichnet, daß** der Querabschnitt (20) der untersten Lage des Stapels an einer Basis lösbar befestigt ist.

5. Nothilfepackung nach Anspruch 4, **dadurch gekennzeichnet, daß** die Basis der Boden (33) eines viereckigen Kastens (30) mit Seitenwandung (31) ist und daß die Seitenwandung (31) einen Ausschnitt (32) zum Durchlaß der Materialbahn (11) aufweist.

6. Nothilfepackung nach Anspruch 5, **dadurch gekennzeichnet, daß** der Kasten (30) einen Klappdeckel (34) aufweist und der Ausschnitt (32) der Klappachse (35) des Klappdeckels (34) gegenüberliegt und daß der Klappdeckel (34) mit einem Leistenteil (36) versehen ist, der den Ausschnitt (32) in der Seitenwandung (31) verschließt.

7. Nothilfepackung nach Anspruch 4, **dadurch gekennzeichnet, daß** die Basis Teil eines Verpakkungsbeutels ist.

## Claims

1. A first-aid kit comprising a flexible elongate material web (11) divided into transverse sections (20-24) having pockets (13) for receiving first-aid articles,
said pockets (13) being provided on both longitudinal halves of the material web and the openings (14) of said pockets (13) extending substantially parallel to the longitudinal center line of the material web (11) and facing each other,
**characterized in**
**that** all transverse sections (20.24) are stacked in zigzag shape.

2. The first-aid kit of claim 1, **characterized in that** the free end of the transverse section (24) of the top layer of the stack is provided with a handle.

3. The first-aid kit of claim 2, **characterized in that** the transverse section (24) of the top layer of the stack has a flap (25) in the material web that is provided with a handle hole (26).

4. The first-aid kit of one of claims 1 to 3, **characterized in that** the transverse section (20) of the bottom layer of the stack may be removably fixed to a base.

5. The first-aid kit of claim 4, **characterized in that** the base is the bottom (33) of a quadrangular box (30) with a side wall (31), and that the side wall (31) comprises a cutout (32) for the passage of the material web (11).

6. The first-aid kit of claim 5, **characterized in that** the box (30) has a hinged cover (34) and the cutout (32) is situated opposite the pivot axis (35) of the hinged cover (34), and that the hinged cover (34) is provided with a strip member (36) closing the cutout (32) in the side wall (31).

7. The first-aid kit of claim 4, **characterized in that** the base is part of a packaging bag.

## Revendications

1. Trousse de premier secours, se composant d'une bande de matériau longue et souple (11), laquelle est divisée en segments transversaux (20 à 24) qui présentent des poches (13) destinées à recevoir les produits de premier secours,
les poches (13) étant agencées sur les deux moitiés de longueur de la bande de matériau et les ouvertures (14) des poches (13) s'étendant de manière pratiquement parallèle à la ligne médiane de la bande de matériau (11) et étant tournées les unes vers les autres,
**caractérisée en ce que**,
tous les segments transversaux (20-24) sont empilés les uns sur les autres en formant des zigzags.

2. Trousse de premier secours selon la revendication 1, **caractérisée en ce que** l'extrémité libre du segment transversal (24) de la couche supérieure de la pile est munie d'une poignée.

3. Trousse de premier secours selon la revendication 2, **caractérisée en ce que** le segment transversal (24) de la couche supérieure de la pile présente une languette (25) ménagée dans la bande de matériau dans laquelle se trouve un trou de préhension (26).

4. Trousse de premier secours selon l'une des revendications 1 à 3, **caractérisée en ce que** le segment transversal (20) de la couche inférieure de la pile est fixé de manière amovible sur une base.

5. Trousse de premier secours selon la revendication 4, **caractérisée en ce que** la base est le fond (33) d'une boîte rectangulaire (30) munie d'une cloison latérale (31) et **en ce que** la cloison latérale (31) présente une découpe (32) pour laisser passer la bande de matériau (11).

6. Trousse de premier secours selon la revendication 5, **caractérisée en ce que** la boîte (30) présente un couvercle rabattable (34) et **en ce que** la découpe (32) est voisine de l'axe de rabat (35) du couvercle rabattable (34) et **en ce que** le couvercle rabattable (34) est muni d'une partie formant bordure (36) qui ferme la découpe (32) dans la paroi latérale (31).

7. Trousse de premier secours selon la revendication 4, **caractérisée en ce que** la base est une partie d'un sachet d'emballage.
